# EUROPÄISCHE PATENTSCHRIFT

(11) **EP 1 426 045 B1**
(45) Veröffentlichungstag und Bekanntmachung des Hinweises auf die Patenterteilung: **27.09.2006**
(21) Anmeldenummer: 02027190.4
(22) Anmeldetag: 05.12.2002
(51) Int. Cl.: A61K 9/48, A61J 3/07, A61K 9/00, A61K 47/42

(54) **Nahtlose gefüllte Kapseln**
Seamless filled capsules
Gélules remplies sans soudure

(43) Veröffentlichungstag der Anmeldung: 09.06.2004
(60) Teilanmeldung: 06116155.0
(73) Patentinhaber: Symrise GmbH & Co. KG, 37603 Holzminden (DE)
(72) Erfinder: Schleifenbaum, Birgit, 37671 Höxter (DE); Voigt, Ines, 37603 Holzminden (DE); Aickele, Frank, 37603 Holzminden (DE)
(74) Vertreter: Eisenführ, Speiser & Partner

(56) Entgegenhaltungen:
- WO-A-00/51574
- WO-A-96/29986
- US-A- 4 517 216
- US-A- 5 300 305
- US-A- 5 370 864
- US-A- 5 780 056
- DATABASE WPI Section Ch, Week 198911 Derwent Publications Ltd., London, GB; Class B07, AN 1989-082882 XP002236113 & JP 01 037259 A (SAN-EI CHEM IND LTD), 7. Februar 1989 (1989-02-07)

## Beschreibung

Die vorliegende Erfindung betrifft sphärische Kapseln mit einem flüssigen Kern und einer diesen Kern umgebenden nahtlosen, festen Hülle; der Kern umfasst dabei einen Aromaanteil und die Hülle umfasst Gelatine. Die Erfindung betrifft auch Verfahren zur Herstellung solcher Kapseln sowie aushärtbare Hüllmischungen (Hüllformulierungen) zur Verwendung bei der Herstellung der erfindungsgemäßen Kapseln. Die erfindungsgemäßen Kapseln zeichnen sich dadurch aus, dass sie ohne weitere Verarbeitung zum Direktverzehr geeignet sind und im Mund gelutscht oder zerkaut werden können, ohne dass die Hülle dabei als störend empfunden wird.

Zur Erzielung von Geschmackseindrücken werden in Lebensmitteln Aromen eingesetzt. Im Sinne der vorliegenden Erfindung sind unter einem Aroma ein einzelner Stoff (Aromastoff) oder eine Mischung aus Stoffen (Aromastoffen) zu verstehen, der bzw. die organoleptische Eigenschaften besitzen. Zu diesen organoleptischen Eigenschaften zählen die Eigenschaft, einer Mischung einen bestimmten Geruch oder Geschmack zu verleihen, und die Eigenschaft, bestimmte Reize hervorzurufen, die über den Trigeminusnerv geleitet und so wahrgenommen werden.

Öle, wie beispielsweise Pflanzenöle und andere Triglyceride, die als Lösungsmittel für das Aroma verwendet werden, und die selbst geruchs- und geschmacksneutral sind, werden nachfolgend nicht als Aromastoffe betrachtet.

Sollen von einem Konsumenten Aromen portionsweise in Form einer Flüssigkeit aufgenommen werden, dann bietet sich die Verwendung einer Kapsel an, bei der ein Kern aus einer aromahaltigen Flüssigkeitsmenge von einer festen Hülle umgeben ist. Hierbei sind insbesondere folgende Probleme und Anforderungen zu beachten:
1. Für eine optimale organoleptischen Wahrnehmung muss die Freisetzung des aromahaltigen, flüssigen Kerns bereits im Mund erfolgen. Sinnvollerweise sollte die Kapsel inklusive der Hülle verzehrfähig sein, da eine Trennung von Hülle und Flüssigkem nach Aufnahme der Kapsel in den Mund problematisch ist.
2. Das Erreichen eines angenehmen Mundgefühls beim Verzehr von Gelatinekapseln ist eine technologische Herausforderung. Insbesondere wird es bislang als nachteilig empfunden, dass die meisten Hüllen von im Handel erhältlichen verzehrfertigen Kapseln im Mund als unangenehmer gummiartiger, zäher Rückstand wahrnehmbar sind. Insbesondere bei großen gefüllten Kapseln (Durchmesser ≥ 4 mm) werden entsprechende Wahrnehmungen gemacht und als besonders negativ empfunden.
3. Beim Verzehr sollte eine Kapselhülle im Mund vorzugsweise (a) haptisch nicht störend wirken, (b) sich gut zerbeißen lassen und (c) sich schnell auflösen. Die freizusetzende bzw. freigesetzte aromahaltige Kernflüssigkeit soll einen sensorischen Effekt mit starkem Impakt im Mund hervorrufen.
4. Gleichzeitig sollte sich die Kapsel gut handhaben lassen, d.h. eine gewisse Mindestgröße aufweisen; bei sphärischen Kapseln wird in vielen Fällen ein Durchmesser ≥ 4 mm bevorzugt.
5. Außerdem sollte die Kapsel zur einfachen Handhabung bei Transport, Lagerung und Gebrauch eine ausreichende Temperaturstabilität besitzen. Wünschenswert ist hierzu, daß die Kapseln auch bei Temperaturen über 30°C nicht verkleben.

Sphärische Kapseln mit einem flüssigen Kern und einer diesen Kern umgebenden festen Hülle sind bekannt, und Kapseln mit einem Durchmesser von mehr als 4 mm können beispielsweise über das Rotary Die Verfahren oder bei den besonders interessanten Kapseln mit nahtloser Hülle über ein Tropfverfahren mit Mehrstoffdüse hergestellt werden (vgl. Bauer, Frömming, Führer; Pharmazeutische Technologie; 1997). Dieses Verfahren wird nachfolgend auch als Mehrstoffdüsen-Verfahren bezeichnet. Bezugnahmen auf das Mehrstoffdüsen-Verfahren sind dabei (soweit sich aus dem Zusammenhang nichts anderes ergibt) auch als Bezugnahmen auf eine Vielzahl verwandter Verfahren zur Herstellung nahtloser Kapseln zu verstehen.

Im Rotary Die Verfahren werden Kapseln mit einer Naht in der Hülle über ein Stanzverfahren mit rotierenden Formwalzen erzeugt, bei dem die Kapselwand aus zwei Gelatinehälften zusammengesetzt und geformt wird, die aus einem Gelatineband ausgestanzt sind. Für die Herstellung von Weichgelatinekapseln nach dem Rotary Die Verfahren werden hohe Anforderungen an die Klimatisierung gestellt. 20 - 30% relative Luftfeuchte bei etwa 22°C müssen in allen Herstellungs- und Verpackungsräumen gewährleistet sein.

Im Mehrstoffdüsen-Verfahren werden Kapseln mit einer nahtlosen Hülle nach einem Tropfverfahren hergestellt. Hierbei werden üblicherweise ein lipophiles Kernmaterial und eine warme Gelatinelösung gleichzeitig durch eine konzentrische Mehrstoffdüse gepumpt, so dass sie in eine kalte lipophile Kühlflüssigkeit, beispielsweise Pflanzenöl, eintropfen. Die Düse kann dabei direkt in die Kühlflüssigkeit eintauchen. Beim Eintropfen nehmen die Kapseln aufgrund der Grenzflächenspannungen Kugelform (sphärische Form) an. Durch die Temperaturabsenkung bei Kontakt mit der Kühlflüssigkeit erstarrt die gelatinehaltige nahtlose Kapselhülle.

US 4,481,157 und US 4,251,195 beschreiben Verfahren und Apparate zur kontinuierlichen Herstellung von nahtlosen Kapseln nach dem Mehrstoffdüsen-Verfahren, bei dem die Düse in die Kühlflüssigkeit eintaucht.

JP 52-148635 beschreibt eine Kapsel mit einem Durchmesser von 0,5 - 4 mm für den Direktverzehr. Die Wanddicke der Kapsel beträgt 50µm - 200µm. Ein Kapseldurchmesser über 4 mm wird nicht beschrieben.

Für eine bessere Handhabung und Portionierung beim Direktverzehr und einen starken Aromaimpakt durch eine einzige Kapsel im Mund ist allerdings ein Kapseldurchmesser von 4 mm und mehr erforderlich.

Je größer eine Kapsel ist, desto schwieriger ist es jedoch, eine dünne, stabile Hülle zu realisieren, da die Stabilität der Kapsel beim Trocknen und beim Transport mit ansteigendem Verhältnis Kapseldurchmesser/Hülldicke stark absinkt. Außerdem ist die Zentrierung des Kerns und das gleichmäßige Umschließen des Kerns durch eine Hülle bei großen Kapseln äußerst problematisch.

US 5,939,097 beschreibt ein Lebensmittel, das Kapseln enthält, die wiederum einen pharmazeutischen Wirkstoff enthalten, wobei das Verhältnis von Hülldicke zu Kapseldurchmesser 0,01 - 0,05 beträgt, bei Kapseldurchmessem von 0,5 - 5 mm. Die Kapseln sind dazu bestimmt, in das Lebensmittel eingearbeitet zu werden und sollen mit diesem ganz verschluckt werden. Die Kapsel soll erst im Magen-Darm Trakt den Kern freigeben. Die Kapselhülle enthält Gelatine oder Agar. Weitere Angaben zur Zusammensetzung der Hülle werden nicht gemacht, Weichmacher werden nicht genannt.

US 5,300,305 beschreibt nahtlose Kapseln mit einem Durchmesser von 2 - 9 mm, die zum Direktverzehr geeignet sind und zur Atemgeruchskontrolle eingesetzt werden. Aktive Substanzen zur Atemgeruchskontrolle sind dabei aus Löslichkeitsgründen in die Hülle der Kapseln eingearbeitet. Die Kapseln sollen eine längere Zeit im Mund verbleiben, damit sich die den Atemgeruch beeinflussende aktive Substanz aus der Hülle lösen und im Mund einen lang anhaltenden Effekt hervorrufen kann. Die Hülldicke liegt im Bereich von 30µm bis 2 mm. Ausführungsbeispiele zeigen Hüllanteile von nicht unter 13 Gew.% und nur geringe Weichmacheranteile (Sorbitol < 10 % bezogen auf die Hülle). Die Kapsel enthält im Kern bis zu 25 Gew.-% Aroma, bezogen auf die Gesamtmasse der Kapsel. Hergestellt werden die Kapseln beispielsweise durch einen Mehrstoffdüsen- Prozess.

Die Kapselhülle ist ersichtlich auf eine langsame Auflösung im Mund hin entwickelt worden, und eigene Untersuchungen haben nun gezeigt, dass sie sich im Anlutschen als eher hart und störend erweist. Nachteilig bei Verwendung der in der US 5,300,305 beschriebenen Kapsel für einen sofortigen starken Aromaeindruck ist dementsprechend insbesondere das negative Mundgefühl, das durch die sich nur langsam auflösenden Hüllreste im Mund hervorgerufen wird. Außerdem ist, wie erwähnt, der Aromagehalt in der Kapsel auf 25 % beschränkt und die Realisierung eines starken Aromaimpakts deshalb nicht möglich.

US 4,935,243 beschreibt eine Weichgelatinekapsel, deren Hülle beim Kauen schnell zerfällt. Die Hülle besteht aus Wasser (15 - 30%), Weichmacher (17,5 - 35 %) und einem geringen Anteil hydrierter hydrolysierter Stärke (5 - 25%). Ausführungsbeispiele zeigen Hülllösungen, deren Trockenmassenanteil bei etwa 75 % liegt.

Als nachteilig wird insbesondere empfunden, dass die Hüllformulierungen der US 4,935,243 aufgrund der hohen Trockenmasse und der dadurch bedingten hohen Viskosität nur für ein Rotary Die Verfahren und nicht für ein Tropfverfahren geeignet sind, d.h. zur Herstellung von Kapseln mit Naht, nicht aber zur Herstellung von nahtlosen Kapseln nach beispielsweise dem Mehrstoffdüsen-Verfahren.

WO 96/29986 beschreibt nahtlose Kapseln mit einem Durchmesser von 2 - 9 mm, die einen pharmazeutischen Wirkstoff gegen Husten enthalten. Die Kapselhüllen sind 30 µm - 500 µm dick und sollen sich innerhalb von 3,5 - 5 Minuten auflösen. Die Kapselhüllen enthalten mindestens 10% Wasser. Ein Verhältnis von Hülldicke zu Kapseldurchmesser wird nicht angegeben.

Nachteilig ist bei den Kapseln gemäß WO 96/29986 insbesondere die angegebene geringe Auflösegeschwindigkeit der Hülle.

US 5620707 beschreibt nahtlose sphärische Kapseln mit einem Durchmesser von 2 - 15 mm und einer Hülldicke von 30µm - 2000 µm für die Anwendung in Getränken, die Aroma, Acesulfam und einen weiteren Süßstoff in bestimmten Verhältnissen im Kern enthalten. Den Ausführungsbeispielen ist zu entnehmen, daß die Hülle über 10% Wasser enthält und als Weichmacher Sorbitol in Mengen über 15 % in der Hülle verwendet wird. Eine Formulierung ganz ohne Weichmacher ist ebenfalls genannt.

Es war eine primäre Aufgabe der vorliegenden Erfindung, sphärische Kapseln der eingangs genannten Art anzugeben, die ohne weitere Verarbeitung zum Direktverzehr geeignet sind und im Mund gelutscht oder zerkaut werden können, ohne dass dabei die Hülle als störend empfunden wird. Die vorstehend angegebenen Probleme und Anforderungen sollten dabei zumindest teilweise, vorzugsweise aber sämtlich gelöst bzw. berücksichtigt sein. Zudem sollte ein in großtechnischem Maßstab praktikables Herstellverfahren für die Kapseln angegeben werden. Zudem sollte auf eine Trocknung in Gegenwart von Trennmitteln verzichtet werden können, die üblicherweise eine nachteilige Trübung der Kapselhülle mit sich bringt.

Eine spezielle (Teil-)Aufgabe der vorliegenden Erfindung war es, eine sphärische nahtlose Kapsel mit einer nur 20 - 200 µm dicken Hülle, einem Durchmesser im Bereich von 4 - 8 mm und einem Verhältnis von Hülldicke zu Kapseldurchmesser im Bereich von 0,004 - 0,04 anzugeben, deren während der Herstellung vor der Trocknung noch feuchte Hülle auch bei Temperaturen von 40°C - 60°C noch formstabil bleibt. Hierdurch sollte sowohl eine höhere Trocknungsgeschwindigkeit (durch mögliche Anwendung höherer Trocknungstemperaturen) als auch eine verbesserte Lagerungs- und Transportfähigkeit realisiert werden.

Erfindungsgemäß wird zur Lösung der gestellten Aufgabe(n) eine sphärische Kapsel mit einem flüssigen Kern und einer diesen Kern umgebenden nahtlosen, festen Hülle angegeben, wobei der Durchmesser der Kapsel im Bereich von 4 - 8 mm liegt, die Dicke der Hülle im Bereich von 20 - 200 µm liegt, das Verhältnis von Hülldicke zu Kapseldurchmesser im Bereich von 0,004 - 0,04 mm liegt, die Hülle 70 - 90 Gew.-% Gelatine und 10 - 30 Gew.-% Weichmacher enthält, bezogen auf die Trockenmasse der Hülle, und der Kern einen Aromaanteil im Bereich von 1 - 100 Gew.-% enthält, bezogen auf die Gesamtmasse des Kerns. Die Kapsel läßt sich nach einem Mehrstoffdüsen- Verfahren herstellen; sie ist ohne weitere Verarbeitung zum Direktverzehr geeignet und kann im Mund gelutscht oder zerkaut werden, ohne dass die Hülle dabei als störend empfunden wird. Bei Wahl eines geeigneten Trocknungsverfahrens (ohne Trennmittel) ist die Kapselhülle glänzend und transparent. Eine Kapsel wird dabei als sphärische Kapsel bezeichnet, soweit das Verhältnis zwischen dem größten und dem kleinsten Durchmesser der Kapsel nicht mehr als 1,2 beträgt. Als Durchmesser einer erfindungsgemäßen Kapsel wird nachfolgend der arithmetische Mittelwert aus dem größten und dem kleinsten Durchmesser der Kapsel bezeichnet.

Für das organoleptische Empfinden ist es besonders vorteilhaft, wenn der Durchmesser der Kapsel im Bereich von 4,5 - 6,5 mm, die Dicke der Hülle im Bereich von 50 - 150 µm und das Verhältnis von Hülldicke zu Kapseldurchmesser im Bereich von 0,01 - 0,03 liegt. Am vorteilhaftesten ist es, wenn der Durchmesser der Kapsel im Bereich von 4,5 - 5,5 mm, die Dicke der Hülle im Bereich von 50 - 90 µm und das Verhältnis von Hülldicke zu Kapseldurchmesser im Bereich von 0,01 - 0,02 liegt

Bevorzugte Ausgestaltungen der erfindungsgemäßen Kapsel ergeben sich aus der nachfolgenden Beschreibung, den Beispielen und den Patentansprüchen.

### HÜLLDICKE:

Für eine schnelle Auflösung der Hülle einer erfindungsgemäßen Kapsel im Mund sollte die Hülldicke so gering wie möglich sein. Bei gleichbleibendem Kern-Hülle-Masse-Verhältnis steigt die Hülldicke mit zunehmendem Kapseldurchmesser stark an.

Die erfindungsgemäßen Kapseln besitzen trotz großer Kapseldurchmesser von 4 - 8 mm eine Hülldicke von nur 20 µm - 200 µm. Dabei liegt das Verhältnis von Hülldicke zu Kapseldurchmesser im Bereich von 0,004 - 0,04. Diese Angaben beziehen sich auf die getrocknete Kapsel. Der Kapseldurchmesser kann dabei mit einer Mikrometerschraube bestimmt werden. Zur Bestimmung der Hülldicke wird von der Kapsel ein Querschnitt angefertigt. Über ein Mikroskop mit Bildauswertung kann die Dicke der Hülle bestimmt werden. Es wird dazu an mehreren Stellen der Kapselhülle deren Dicke gemessen und der mathematische Mittelwert für die Hüllstärke bestimmt.

Bei erfindungsgemäß gewählter Zusammensetzung der Hülle (siehe dazu unten) sind eine hohe Prozeß- und Transportstabilität, eine ausreichende Elastizität, eine gute Zerbeißfähigkeit sowie ein ausreichend hohes Auflösevermögen im Mund gewährleistet.

### ZUSAMMENSETZUNG DER HÜLLE:

### Viskosität der Hüllmischung / Gelierpunkt:

Bei der Ausformung einer erfindungsgemäßen Kapsel über ein Mehrstoffdüsen-Verfahren sind die Viskosität und das Gelierverhalten der aushärtbaren Hüllmischung besonders zu beachten. Eine zu niedrige Viskosität oder ein zu niedriger Gelierpunkt verhindern eine ausreichend feste Kapselhülle im nassen Zustand. Entsprechende Kapseln würden durch die weiteren Prozessschritte wie z.B. Zentrifugieren mechanisch zerstört. Eine zu hohe Viskosität und ein zu hoher Gelierpunkt verhindern andererseits eine korrekte Kapselausformung und verursachen zudem eine unerwünscht starke Satellitenbildung.

Die Viskosität bevorzugter Hüllmischungen zur Herstellung erfindungsgemäßer Kapseln wurde mit einem Rheometer CVO 120 (Fa. Bohlin Instruments GmbH, Pforzheim) bestimmt. Als Messsystem diente ein Platte-Platte-System mit einem Plattendurchmesser von 50 mm. Es wurde in Rotation gemessen. Die Scherrate betrug 50 s⁻¹, der Spalt wurde auf 500 µm eingestellt. Es wurde isotherm gemessen, die Temperatur lag bei 80°C.

Bevorzugte Hüllmischungen besitzen bei 80°C eine Viskosität im Bereich von 30 mPas bis 300 mPas, bevorzugt von 40 mPas bis 150 mPas, besonders bevorzugt von 50 mPas bis 90 mPas.

Der Gelierpunkt bevorzugter Hüllmischungen zur Herstellung erfindungsgemäßer Kapseln wurde ebenfalls mit einem Rheometer CVO 120 (Fa. Bohlin Instruments GmbH, Pforzheim) bestimmt. Als Messsystem diente ein Platte-Platte-System mit einem Platten Durchmesser von 50 mm. Es wurde in Oszillation gemessen. Die Frequenz betrug konstant 1 Hz, der Spalt wurde auf 500 µm eingestellt, die Temperatur wurde von 80°C auf 10°C mit einem Gradienten von 5°C/min abgesenkt. Als Gelierpunkt, dem Sol/Gel Übergangspunkt, wurde die Temperatur abgelesen, bei der Viskositäts- oder Speichermodul G' gleich dem Elastizitäts- oder Verlustmodul G" ist. (Thomas Mezger, Das Rheologie Handbuch, 2000).

Die Gelierpunkte bevorzugter erfindungsgemäßer Hüllmischungen liegen zwischen 15°C und 60°C, vorzugsweise zwischen 20°C und 40°C, besonders bevorzugt zwischen 25°C und 35°C.

### Gelatine:

Die Hülle der erfindungsgemäßen Kapseln enthält Gelatine und Weichmacher. Qualität und Menge der Gelatine und des Weichmachers haben Auswirkung auf die Löslichkeitskinetik der Hülle im Mund.

Zur Kapselausformung werden für die Hülle vorzugsweise wäßrige Lösungen mit 10 - 40 Gew.-%, bevorzugt 15 - 30 Gew.-%, besonders bevorzugt 18 - 25 Gew.-% Gelatine verwendet.

Die in erfindungsgemäßen Kapseln verwendete Gelatine wird in vielen Fällen durch partielle Hydrolyse von collagenhaltigem Material von Tieren, wie beispielsweise Schweinen, Rindern, Fischen, Geflügel gewonnen. Typ A Gelatine wird durch sauren Aufschluß meist von Schweine- oder Fischhäuten gewonnen, während Typ B Gelatine durch alkalischen Aufschluß meist von Rinderknochen und -häuten gewonnen wird.

Zur Kennzeichnung der Gallertfestigkeit von Gelatine wird der Begriff Bloom verwendet. Bei der Bestimmung des Bloomwertes wird ein Stempel eines Bloom-Gelometers oder Texture Analysers von 12,7 mm (0,5 Zoll) Durchmesser 4 mm tief in ein 6,67 % iges Gelatinegel eingedrückt, das vor der Messung bei 10°C 18 Stunden gealtert wurde. Die Angabe erfolgt in "Bloom", entsprechend dem Gewicht in Gramm, das auf dem Stempel zur Erzielung der Eindrucktiefe lastet. (vgl. Schormüller, Handbuch der Lebensmittelchemie, Band III, 1968 und British Standard Method for Sampling and Testing Gelatine (BS757; 1975)).

Zur Herstellung erfindungsgemäßer Kapseln wird bevorzugt eine Gelatine mit einem Bloom-Wert von über 200, besonders bevorzugt mit einem Bloom-Wert von 240-300 eingesetzt, und zwar vorzugsweise eine Typ A Gelatine. Damit wird eine ausreichende Stabilität der Hülle während der Kapselherstellung und während des Transports trotz geringer Dicke der Kapselhülle ermöglicht.

Auch Gelatinequalitäten, die aus Rind, Geflügel oder Fisch gewonnen werden, sind für die Herstellung der erfindungsgemäßen Kapsel geeignet. Zu beachten ist dabei in jedem Fall, wie bereits erwähnt, daß die Viskosität und das Gelierverhalten richtig eingestellt werden. Als Fischgelatine können sowohl Qualitäten aus Kaltwasserfischen als auch Qualitäten aus Warmwasserfischen verwendet werden. Es können auch Mischungen verschiedener Gelatinequalitäten eingesetzt werden. Einzelheiten sind den Beispielen zu entnehmen.

Eine hohe Prozeßstabilität einer Kapsel bei gleichzeitig schneller Löslichkeit der Kapselhülle im Mund zu erreichen, ist eine besondere technische Aufgabe. Einerseits ist es nämlich für den Herstellungsprozeß der Kapsel und die Lagerung vorteilhaft, eine Hüllformulierung zu wählen, die der Kapsel eine besonders hohe mechanische Festigkeit gibt, schnell ein festes Gel bildet, das nach dem Trocknen möglichst hart ist und wenig Wasser aufnimmt. Andererseits ist es für eine gutes Mundgefühl beim Verzehr der Kapsel von Vorteil, wenn die Hülle schnell Wasser aufnimmt, weich und flexibel ist, und sich schnell löst.

Überraschend wurde gefunden, daß bei Verwendung eines Gemisches aus einer hydrolysierten Gelatine mit Bloomwert 0 und einer hochbloomigen Gelatine mit einem Bloomwert von 200 und darüber (vorzugsweise einem Bloomwert im Bereich von 240 - 300) diese besondere technische Aufgabe gelöst werden kann. Die hochbloomige Gelatine bildet dabei vermutlich ein festes Netzwerk, das für die Prozeßstabilität wichtig ist. Die hydrolysierte 0-bloomige Gelatine besetzt vermutlich Zwischenräume in diesem Netzwerk und führt im Mund zu einer schnelleren Wasseraufnahme und dadurch Löslichkeit der gesamten Hülle. Vgl. Beispiel 26 und 27 unten.

Hydrolysierte 0-bloom Gelatine besitzt keine Gelierkraft und ist in Wasser bei 20°C leicht löslich. Die Polypeptidketten sind in dieser 0 bloom Gelatine durch Säure oder enzymatische Hydrolyse sehr stark abgebaut. Sie wird daher auch bisher nicht zur Bildung von Kapselhüllen eingesetzt, sondern lediglich z. B. als Nährstoff (Proteinquelle), als Emulgator, oder auch zum Klären von Wein verwendet.

Zur Lösung der besonderen technischen Aufgabe eignen sich vorzugsweise HüllMischungen aus (a) hydrolysierter 0-bloom Gelatine, die aus beliebigen Tierarten gewonnen wurde, mit (b) Gelatine, die einen Bloomwert von ≥ 200 besitzt, wobei der Anteil der hydrolysierten 0-bloom Gelatine vorzugsweise im Bereich von 0,5 - 90 Gew.-% liegt, bezogen auf die Trockenmasse der Hülle.

Der Gelierpunkt der Mischungen wird dabei maßgeblich vom Gelierpunkt des hochbloomigen Gelatineanteils bestimmt (siehe die beigefügte Tabelle "Gelierpunkte").

Die Flexibilität eines aus einer Hüll-Mischung hergestellten Films ist im übrigen überraschend hoch, wenn die Mischung 0-bloomige Gelatine umfasst (siehe die beigefügte Tabelle "Gelierpunkt").

Eine weitere Möglichkeit, die oben genannte besondere technische Aufgabe zu lösen, besteht in der Mischung von bestimmten niedrigbloomigen (Bloomwert < 200) Fischgelatinen mit hochbloomiger Gelatine (Bloomwert ≥ 200).

Je niedriger der Bloomwert einer Gelatine, desto niedriger sind im allgemeinen der Gelierpunkt, die Viskosität sowie die mechanische Stabilität des feuchten erstarrten Gels. Mischungen aus Gelatine mit niedrigem Bloomwert und mittlerem Bloomwert zur Erzielung einer verbesserten Löslichkeit der Kapselhülle im Mund sind bekannt. US 6,258,380 beschreibt derartige Hüllen. Hierbei wurde allerdings keine über den Bloomwert hinausgehende Spezifizierung der Gelatine vorgenommen.

Überraschend wurde nun gefunden, dass bei Verwendung von Fischgelatine als Gelatine-Anteil mit niedrigem Bloomwert (< 200) und gleichzeitiger Verwendung einer hochbloomigen Gelatine mit Bloomwert von 200 und darüber, eine weiter verbesserte Löslichkeit im Mund erzielt werden kann. Dies wird vermutlich durch den niedrigeren Gelierpunkt von Fischgelatinen (unter 28 °C) im Vergleich mit Schwein-, Rind-, und Geflügelgelatinen (etwa 28 - 40 °C) bewirkt.

Insbesondere eignen sich Fischgelatinequalitäten mit einem Gelierpunkt < 20° C und Qualitäten, die aus Kaltwasserfischen, beispielsweise Kabeljau, hergestellt werden, da deren Gelierpunkte mit etwa 10 - 20 °C noch unter denen von Gelatinen aus Warmwasserfischen (Gelierpunkt etwa 20 - 28 °C), wie beipielsweise aus Karpfen, liegen. Zu den Kaltwasserfischen zählen hierbei alle Fischarten, die sich vorwiegend in Gewässern mit Temperaturen von 18°C und darunter aufhalten. Untersuchungen von Choi und Regenstein (Journal of Food Science Vol.65, No.2, 2000) sowie eigene Untersuchungen (siehe beigefügte Tabelle "Gelierpunkte") bestätigen die niedrigeren Gelierpunkte von Fischgelatinen gegenüber Schwein-, Rind-, Hühnergelatine bei in etwa vergleichbaren Bloomwerten.

Vermutlich ist für die bei Fischgelatinequalitäten niedrigeren Gelierpunkte die Proteinzusammensetzung von Bedeutung. Die Anteile der Aminosäuren Prolin und Hydroxyprolin sind bei Fischgelatinen, und zwar insbesondere bei Kaltwasserfischgelatinen im Vergleich zu Schweine-, Rinder-, und Geflügelgelatine, deutlich niedriger. Hydroxyprolin und Prolin spielen eine bedeutende Rolle bei der Vernetzung der Proteinhelices untereinander. In Wasser kommt es vermutlich zur Auffaltung der Helices, Wasser kann eingelagert werden, und die Löslichkeit steigt. Die Temperatur, bei der diese Auffaltung stattfindet, hängt vom Hydroxyprolin- und Prolingehalt ab. Je niedriger der Gehalt, desto niedriger die Temperatur, bei der die Gelatine in Lösung geht.

Eine niedrige Geliertemperatur und eine niedrige Löslichkeitstemperatur ist für eine gute Löslichkeit im Mund vorteilhaft.

Durch Verwendung von Fischgelatine mit einem Bloomwert von unter 200 als alleinige Gelatineart in der Hülle ist eine ausreichende Prozeßstabilität der Kapsel in der Regel nicht zu erreichen. Die Gelfestigkeit der noch feuchten Kapselhüllen ist für einen weiteren Prozeßverlauf häufig nicht ausreichend. Die Kapseln sind häufig mechanisch zu instabil.

Hingegen wird durch die Beimischung einer solchen Gelatine als schnelllöslicher Füllstoff zu einer hochbloomigen Gelatine, die ein prozeßstabiles Gerüst bilden soll, die besondere technische Aufgabe gelöst. Dabei haben sich Mischungen aus Fischgelatinen mit Bloomwerten unter 200 und hochbloomigen Schweine-, Rinder-, oder Geflügelgelatinen mit Bloomwert über 200 als vorteilhaft erwiesen. Bevorzugt sind Anteile an Fischgelatine (unter 200 bloom) von 0,5 - 50 Gew. %, bezogen auf die Trockenmasse der Hülle. Besonders bevorzugt sind dabei Gelatinequalitäten aus Kaltwasserfischen.

Der Gelierpunkt der Mischungen wird dabei maßgeblich vom Gelierpunkt des hochbloomigen Gelatineanteils bestimmt (siehe die beigefügte Tabelle "Gelierpunkte").

### Weichmacher:

Als Weichmacher können insbesondere Polyole wie beispielsweise Sorbitol, Glycerin, Propylenglykol, Lactitol, hydrierte Stärkehydrolysate, Trehalose eingesetzt werden. Weichmacheranteile verbessern die Verzehrseigenschaften einer Kapsel, indem Sie die Härte der Kapselhülle herabsetzen und die Löslichkeit im Mund verbessern. Weichmacher fördern zudem die Flexibilität der Hülle und damit die Stabilität bei der Kapseltrocknung und beim Transport.

Bevorzugte Weichmacheranteile für die erfindungsgemäßen Kapseln liegen nicht über 30 Gew.-%, bezogen auf die Gesamttrockenmasse der Hülle. Höhere Mengen Weichmacher erschweren die Trocknung der Kapseln und machen außerdem Verpackungen erforderlich, die die Luftfeuchtigkeit ausschließen.

Bevorzugt werden Weichmacher mit einem Anteil von 10 - 30 Gew.-%, besonders bevorzugt von 15 - 20 Gew.-% in der Hülle, bezogen auf die Trockenmasse der Hülle, eingesetzt. Bevorzugt umfasst der Weichmacher ein oder mehrere Polyole, vorzugsweise ausgewählt aus der Gruppe, die aus Glycerin, Propylenglykol, Sorbitol und Maltitol besteht. Glycerin wird als Weichmacher bevorzugt.

Anteile Weichmacher über 30 Gew.-% erschweren die Trocknung erfindungsgemäßer Kapseln und machen häufig die Verwendung von Anticakingmitteln wie Kieselsäure notwendig. Da die erfindungsgemäßen Kapseln aber aus optischen Gründen in der Regel eine transparente, glänzende Hülle aufweisen sollen, ist die Verwendung von Kieselsäure nicht erwünscht.

Anteile Weichmacher unter 10 Gew.-% lassen die Kapselhülle einer erfindungsgemäßen Kapsel zunehmend spröde werden

Versuche haben gezeigt, daß im Falle von Sorbitol bereits Anteile von über 15 Gew.-% in der Hülle zu Problemen bei der Kapseltrocknung führen können und dann die unerwünschte Verwendung eines Anticakingmittels notwendig machen würde.

### WEITERE (OPTIONALE) BESTANDTEILE DER KAPSELHÜLLE:

### Süßstoffe / Farbstoffe / Wasser:

Zusätzlich zu Gelatine und Weichmacher kann die Hülle einer erfindungsgemäßen Kapsel Süßstoffe wie beispielsweise Sucralose, Aspartam, Acesulfam K, Na-Saccharin, Thaumatin, Neohesperidin, oder Mischungen hieraus, sowie wasserlösliche Lebensmittelfarbstoffe enthalten.

Während des Herstellungsprozesses werden die Kapseln getrocknet. Eine gewisse Restmenge Wasser verbleibt hierbei gebunden im Gelatinenetzwerk. Je nach Umgebungsfeuchte wird sich ein Wassergehalt in der Kapselhülle im Gleichgewicht einstellen. Die Gleichgewichtsfeuchte typischer erfindungsgemäßer Kapseln liegt bei 20°C und 50% relativer Luftfeuchtigkeit im Bereich von etwa 8 - 10 Gew.-% Wasser, bezogen auf die Gesamtmasse der Kapselhülle.

### Hydrokolloide / Gellan Gum:

Zugaben von Hydrokolloiden zur Gelatine beeinflussen die Löslichkeit und damit die Aufnahme von Wasser sowie die Temperaturstabilität der gebildeten Gele.

Als Beimischung zur Gelatine einer Hüllmaterialmischung zur Herstellung einer erfindungsgemäßen Kapsel kann insbesondere das Hydrokolloid Gellan Gum vorteilhaft verwendet werden. Gellan Gum ist ein gelbildendes Polysaccharid, das mit Hilfe von Mikroorganismen durch Fermentation hergestellt wird

US 4517216 *(Merck)* beschreibt bereits Mischungen aus Gelatine und Gellan Gum. Durch einen Gellan Gum Anteil von 16% - 83 % bezogen auf die Summe aus Gellan Gum- und Gelatinemenge wird eine hohe Gelstärke der Hülle durch einen synergistischen Effekt erreicht. Beschrieben ist außerdem, daß nur die deacylierten und teilweise deacylierten Formen von Gellan Gum diesen Effekt hervorrufen, nicht aber das native Gellan Gum.

JP 4027352 *(Fuji)* beschreibt Weichgelatinekapseln, die nach dem Rotary Die Prozeß hergestellt werden, und die sich erst im Darmtrakt auflösen. Die Veränderung der Löslichkeit der Hülle wird dabei durch die Zugabe von Hydrokolloiden, die mit Calciumionen ein Gel bilden, wie beispielsweise Gellan Gum, hervorgerufen. Nachteilig ist hierbei die Zugabe von Calciumionen zur Hülllösung. Eine Herstellung von nahtlosen Kapseln nach dem Mehrstoffdüsen-Verfahren ist mit dieser Hüllmischung nicht möglich, da durch die Calciumionen die Viskosität und der Gelierpunkt der Hülllösung zu hoch sind .

JP 1037259 *(San Ei)* beschreibt Gelatinekapseln mit einem Gellan Gum Anteil in der Hülle zum Erreichen einer verbesserte Hüllstärke. Dabei sind 0,08% - 2,4 % Gellan Gum bezogen auf die Summe aus Gellan Gum- und Gelatinemenge in der Hülle enthalten. Die Trockenmasse der Hülle beträgt mindestens 50 %. Aufgrund der dadurch hervorgerufenen hohen Viskosität ist diese Formulierung nicht für einen Mehrstoffdüsen- Prozeß für die Herstellung von nahtlosen Kapseln geeignet.

JP 63170310 *(San Ei)* beschreibt Kapseln mit Hüllen aus Gellan Gum und anderen Hydrokolloiden, u.a. Gelatine. Dabei liegt der Anteil des Gellan Gum an der Gesamtmasse der Hydrokolloide im Bereich von 50 - 80 Gew.-%. Ziel ist es, eine möglichst hohe Geliertemperatur und Geliergeschwindigkeit zu erzielen.

Die US 2002/0024678 A1 beschreibt Gelatine-Zusammensetzungen für zweiteilige Kapseln, wobei Gellan Gum als Bestandteil eines Abbindesystems (setting system) eingesetzt werden kann. Die in den Beispielen beschriebenen zweiteiligen Kapseln sind Hartkapseln mit einem Auflösevermögen, das für die Zwecke der vorliegenden Erfindung nicht akzeptabel wäre.

Nachteilig an den vorstehend wiedergegebenen Ausgestaltungen ist, daß sie nicht auf das Verfahren zur Herstellung von nahtlosen Weichgelatinekapseln nach dem Mehrstoffdüsen- Verfahren abgestimmt sind. Soll zur Herstellung einer erfindungsgemäßen Kapsel das Mehrstoffdüsen- Verfahren mit eingetauchter Düse angewandt werden, ist es bei der Verwendung von Gellan Gum im Hüllmaterial essentiell, die korrekte Auswahl des Gellan Gums Typs und der Gellan Gum Menge zu treffen, damit die Hülle nicht bereits erstarrt, bevor die Kapselbildung vollständig erfolgt ist. Außerdem darf die Viskosität der Hülllösung nicht zu hoch sein.

Zur Erzielung einer erhöhten Temperaturstabilität wird der Hüllmischung zur Herstellung einer erfindungsgemäßen Kapsel vorteilhafterweise Gellan Gum zugegeben; hierdurch steigt die Erweichungstemperatur der Hülle deutlich an und auch die Geliertemperatur der Mischung ist deutlich erhöht. Für die Herstellung erfindungsgemäßer Kapseln nach dem Mehrstoffdüsen-Verfahren mit eingetauchter Düse sollte die Geliertemperatur nicht über 50°C und die Viskosität der Hüllösung bei 80°C nicht über 300 mPas liegen, andernfalls ist eine Kapselbildung erschwert oder nicht erreichbar. Daher müssen Gellan Gum - Art und -Menge besonders gezielt ausgewählt werden.

Es gibt hoch- und niedrig acylierte Gellan Gum Qualitäten. Zur Herstellung erfindungsgemäßer Kapseln wird vorzugsweise ein niedrig acyliertes Gellan Gum, bevorzugt die Qualität KELCOGEL F von Kelco, einer Division von Merck & Co, eingesetzt. Mit niedrig acylierten Gellan Gum Qualitäten können harte, transparente Gele erzielt werden.

Mit einer hoch acylierten Gellan Gum Qualität, wie beispielsweise KELCOGEL LT100 von Kelco, ist die Herstellung erfindungsgemäße Kapseln im Mehrstoffdüsen-Prozeß problematisch, da sich bei der Kapselausformung die Kapseln aufgrund einer hoher Elastizität der Hülle nicht störungsfrei von der koaxialen Düse ablösen. Außerdem werden unerwünscht trübe und sehr hochelastische weiche Gele erzeugt.

In einer bevorzugten wäßrigen Hüllmischung (Hülllösung) zur Herstellung einer erfindungsgemäßen Kapsel macht Gelatine mit > 15 Gew.-%, bezogen auf die Gesamtmasse der Hüllösung, den Hauptanteil an der Gesamtmasse eingesetzter Hydrokolloide aus. Gellan Gum wird daneben mit einem Anteil von maximal 0,6 Gew.-%, vorzugsweise einem Anteil im Bereich zwischen 0,2 und 0,5 Gew.-% eingesetzt.

Höhere Anteile an Gellan Gum setzen die Viskosität der Hülllösung bei der Kapselausformung stark herauf und die Löslichkeit der getrockneten Kapselhülle im Mund stark herab, was nicht erwünscht ist.

Geringere Anteile an Gellan Gum sind für eine verbesserte Temperaturstabilität der getrockneten Kapsel nicht mehr sonderlich wirksam.

Die Anwesenheit von Gellan Gum führt in der Hülle einer erfindungsgemäßen Kapsel zur Bildung eines festen Netzwerkes, das sich im feuchten Zustand auch bei 40 - 60°C in der Regel nicht löst. Dieses Netzwerk soll als festigendes Element nur einen vergleichsweise geringen Anteil der Hülle ausmachen. Die Anteile unvernetzter Gelatine und weiterer Zutaten wie beispielsweise Weichmacher, sollen sich hingegen im Mund besonders schnell lösen.

Bevorzugt sind erfindungsgemäße Kapseln, die Gellan Gum in einem Bereich von 0,4 - 3 Gew.-%, bevorzugt von 0,8 - 2 Gew.-% enthalten, bezogen auf die Trockenmasse der Hülle. Bei Anteilen von 70 - 90 Gew.-% Gelatine, bezogen auf die Trockenmasse der Hülle, ergibt sich ein bevorzugtes Massenverhältnis von Gellan Gum zu Gelatine im Bereich von 1 : 23 bis 1 : 230, bevorzugt von 1 : 35 bis 1 : 115.

Der Gelatineanteil kann dabei insbesondere auch Anteile 0-bloomiger Gelatine und/oder niedrigbloomiger Fischgelatine enthalten (siehe dazu oben).

### KERNFLÜSSIGKEIT:

Bei Herstellung einer erfindungsgemäßen Kapsel nach dem Mehrstoffdüsen-Verfahren ist die Kernflüssigkeit hydrophob und in der Lage, mit wäßrigen Lösungen ein Zweiphasensystem zu bilden.

Beim Verzehr der erfindungsgemäßen großen Kapseln (Kapseldurchmesser im Bereich von 4 - 8 mm, Dicke der Hülle im Bereich von 20 - 200 µm) gelangt eine relativ große Flüssigkeitsmenge direkt in den Mund. Dabei soll ein möglichst starker sofortiger Aromaeindruck hervorgerufen werden.

Bevorzugt werden deshalb für die Kernflüssigkeit Mischungen aus Aromen mit Pflanzenölen oder Triglyceriden eingesetzt. Die Mischung stellt vorzugsweise bei Raumtemperatur und bevorzugt auch noch bei 10°C eine klare Lösung dar. Beispielsweise eignen sich synthetische und natürliche Aromastoffe und deren Mischungen sowie auch Oleoresine oder Extrakte von Pflanzen, Blättern, Blüten, Früchten und dergleichen, sowie deren Kombinationen. Bevorzugt werden Aromen aus der Reihe Pfefferminzöl, Spearmintöl, Eukalyptusöl, Zimtöl, Cassiaöl, Anisöl, Bittermandelöl, Nelkenöl, Citrusöle, fruchtige Aromenkompositionen der Geschmacksrichtungen wie Apfel, Birne, Pfirsich, Traube, Erdbeere, Himbeere, Kirsche, Ananas, sowie Einzelkomponenten wie Menthol, Menthon, Menthylacetat verwendet.

### Aromaanteil im flüssigen Kern:

Der Aromaanteil in der Kernflüssigkeit richtet sich insbesondere nach der Kapselgröße und der Aromaintensität und reicht erfindungsgemäß von 1 bis 100 %, bezogen auf die Gesamtmasse des flüssigen Kerns. Bevorzugt ist allerdings ein Aromaanteil im flüssigen Kern im Bereich von 5 -90 Gew.-%, vorzugsweise 30 -80 % Gew.-%, bezogen auf die Gesamtmasse des flüssigen Kerns.

### Süßstoffe im flüssigen Kern (optional):

Der Kernflüssigkeit einer erfindungsgemäßen Kapsel können auch Süßstoffe zugesetzt werden, gegebenenfalls unter Einsatz von Lösungsvermittlern. Da die Kernflüssigkeit bestimmungsgemäß im Mund in direkten Kontakt mit den Zähnen kommt, ist es von Vorteil, wenn von der Kernflüssigkeit keine pH-absenkende Wirkung ausgeht. Andernfalls wäre eine Schädigung des Zahnschmelzes nicht auszuschließen.

Es wurde nun in eigenen Untersuchungen gefunden, daß Thaumatin, Neohesperidin und Miraculin (sowie deren Mischungen) als Süßstoffe in der Kernflüssigkeit besonders geeignet sind, und den pH-Wert nicht nachteilig beeinflussen. Aus Löslichkeitsgründen wird Thaumatin besonders bevorzugt.

Es wurde auch gefunden, daß hingegen andere grundsätzlich zum Einsatz geeignete Süßstoffe wie beispielsweise Saccharinsäure oder Acesulfam K, den pH-Wert der Wasserphase absenken, und daher nicht in größeren Konzentrationen eingesetzt werden sollten, wenn die pH-Entwicklung im Mund tolerabel bleiben soll.

Zur Ermittlung des pH-Einflusses wurden 20 ml Wasserphase 5 Minuten lang unter Rühren bei 25°C in Kontakt mit 5 ml der Süßstoffe enthaltenden Kernflüssigkeit gebracht. Nach Phasentrennung wurde der pH-Wert der wäßrigen Phase gemessen. Bei Einsatz von Saccharinsäure oder Acesulfam K wurde der pH der Wasserphase auf unter 4 abgesenkt.

### Öle im flüssigen Kern:

Als Öle zur Verdünnung eingesetzter Aromen eignen sich insbesondere fraktionierte Kokosöle, die hauptsächlich Fettsäurereste C6-C8 aufweisen. Diese Öle zeichnen sich durch ihre Geschmacksneutralität sowie durch ihre gute Oxidationsstabilität aus.

### Weitere Bestandteile des flüssigen Kerns (optional) :

Der Kernflüssigkeit können farbgebende Substanzen, Vitamine und/oder Pflanzenextrakte zugesetzt werden.

### WEITERE, INSBESONDERE PHYSIKALISCHE KAPSELEIGENSCHAFTEN:

### Härte:

Erfindungsgemäße Kapseln besitzen vorzugsweise eine Härte von 1000 - 4000 g. Bevorzugt werden Kapseln, die, z.B. bei einem Durchmesser von 5 mm, eine Härte von 1500 - 3500 g aufweisen.

Härtere Kapseln verursachen üblicherweise ein unangenehmes Mundgefühl, weichere Kapseln verursachen Schwierigkeiten beim Transport, da sie mechanisch nicht stabil sind.

Die Härte der Kapseln wird dabei mit einem Texture Analyser bestimmt, beispielsweise mit einem TA-XT2i der Firma Stable Micro Systems. Dabei wird ein Stempel mit einem Durchmesser von 2 mm mit einem konstanten Vorschub von 0,5 mm/sec auf eine Kapsel soweit abgesenkt, bis die Hülle dieser Kapsel bricht. Als Härte der Kapsel wird das Gewicht in g bezeichnet, das am Bruchpunkt auf der Kapsel lastet.

Zur Härte erfindungsgemäßer Kapseln vergleiche die Beispiele.

### Auflösevermögen:

Die Hülle bevorzugter erfindungsgemäßer Kapseln löst sich im im Mund in weniger als 60 Sekunden, bevorzugt in weniger als 45 Sekunden auf. Bestimmt werden kann die Auflösegeschwindigkeit sensorisch, wobei auch die mechanischen Einwirkungen beim Bewegen der Kapsel im Mund berücksichtigt werden.

### Zum Auflösungsvermögen erfindungsgemäßer Kapseln vergleiche die Beispiele

### Aussehen:

Erfindungsgemäße Kapseln sind kugelförmig (sphärisch). Mit dem Mehrstoffdüsen- Verfahren lassen sich erfindungsgemäße Kapseln herstellen. Das Verhältnis zwischen dem größten und dem kleinsten Durchmesser einer erfindungsgemäßen sphärischen Kapsel beträgt nicht mehr als 1,2 , bevorzugt nicht mehr als 1,1. Bei einem größeren Verhältnis wird die Schalendicke ungleichmäßig und die Kapsel mechanisch labil.

Die Hülle einer erfindungsgemäßen Kapsel ist vorzugsweise transparent und glänzend; daher sollte auf den Einsatz von Trennmitteln bei der Trocknung oder Konfektionierung nach der Trocknung verzichtet werden. Zum Erzielen einer transparenten Hülle sollte auch auf jegliche Zusätze zur Hüllmischung verzichtet werden, die die Hülle trüben, auch wenn die Zusätze positive sonstige Eigenschaften besitzen und beispielsweise die Löslichkeit der Hülle im Mund positiv beeinflussen würden, so wie beispielsweise Cellulosen.

### BEISPIELE:

Nachfolgend werden bevorzugte Ausgestaltungen der Erfindung anhand von Beispielen näher erläutert:

### Beispiele 1-29: Verfahren zur Herstellung erfindungsgemäßer Kapseln - Allgemeine Verfahrensvorschrift (Mehrstoffdüsen-Verfahren mit eingetauchter Düse)

Die in der beigefügten Tabelle Beispiele 1 - 29" angegebenen Bestandteile für die Hüllmischung werden zusammengegeben und im Wasserbad auf 80°C erhitzt, bis eine klare, im wesentlichen luftblasenfreie Lösung entstanden ist. Vorzugsweise werden Lösungen mit Trockenmasseanteilen von 20 - 40 Gew. % verwendet.

Die Kernflüssigkeit wird bei 10 - 20 °C bereitgestellt.

Hüllflüssigkeit und Kernflüssigkeit werden über ein Pumpensystem einer konzentrischen Zweistoffdüse zugeführt. Dabei wird die Leitung für die Hüllflüssigkeit auf 60 - 80°C gehalten. Die konzentrische Zweistoffdüse taucht in ein Flüssigkeitsbad ein, das mit Pflanzenöl gefüllt ist. Die Temperatur dieses Ölbads beträgt ca. 14°C.

Der aus der Düse in das Ölbad austretende Flüssigkeitsstrahl zerfällt, unterstützt durch zusätzliche Schwingungsanregung der Flüssigkeit, zu Einzeltropfen, bei denen es sich um nahtlose Kapseln aus Kern und Hülle handelt.

Die noch nassen Kapseln werden durch Zentrifugieren vom anhaftenden Öl getrennt und anschließend unter ständiger Bewegung in einem trockenen Luftstrom getrocknet. Es können übliche Wirbelbetttrockner oder Trommeltrockner verwendet werden. Voraussetzung für einen guten Trocknungserfolg ist, daß die Kapseln durch Rotation oder durch Luftverwirbelung in Bewegung gehalten werden können. In manchen Fällen ist hierzu der Einsatz eines Fließhilfsmittels ratsam.

Die Verwendung eines Fließhilfsmittels ist jedoch in den meisten Fällen nicht erwünscht. Eine transparente und glänzende Hülle wird nämlich erreicht, wenn die Zusammensetzung der Hüllmischung so gewählt wird, daß auf ein Fließhilfsmittel wie beispielsweise Kieselsäure während der Trocknung verzichtet werden kann, und es trotzdem nicht zum Verkleben der Kapseln kommt

### Beispiele 1 -29 :

### Zusammensetzung und Verzehrseigenschaften der getrockneten Kapseln.

**Optimierung des Weichmachertyps / Weichmachermenge / Hüllanteil**

| **Nr.** | **Kapsel durchmesser** | **Hülldicke** | **Verhältnis Hülldicke/ Kapseldurchmesser** | **Anteil Hülle** | **Anteil Kern** | **Zusammensetzung Hülle** | **Zusammensetzung Kern** | **Härte [g]** | **Auflösen im Mund** |
|---|---|---|---|---|---|---|---|---|---|
| | **[mm]** | **[µm]** | | **[Gew. %]** | **[Gew.%]** | | | | **[sec]** |
| 1x | 5 | 140 | 0,028 | 15 | 85 | 80 % Schweine-Gelatine 260 bloom | 20 % Erdbeeraroma | > 6000 | 90 sec |
| | | | | | | 10 % Glycerin | 80 % Pflanzenöl | | |
| | | | | | | 10% Wasser* | | | |
| 2x | 5 | 88 | 0,018 | 12 | 88 | 80 % Schweine-Gelatine 260 bloom | 30 % Pfefferminzaroma | 4860 | 55 sec |
| | | | | | | 10 % Glycerin | 70 % Pflanzenöl | | |
| | | | | | | 10 % Wasser* | | | |
| | | | | | | 0,04 % Thaumatin | | | |
| 3x | 5 | 80 | 0,016 | 10 | 90 | 80 % Schweine-Gelatine 260 bloom | 30 % Pfefferminzaroma | 4570 | 55 sec |
| | | | | | | 10 % Glycerin | 70 % Pflanzenöl | | |
| | | | | | | 10 % Wasser* | | | |
| 4x | 5 | 70 | 0,014 | 7,5 | 92,5 | 80 % Schweine-Gelatine 260 bloom | 20 % Erdbeeraroma | 3290 | 50 sec |
| | | | | | | 10 % Glycerin | 80 % Pflanzenöl | | |
| | | | | | | 10 % Wasser* | | | |
| 5x | 5 | 50 | 0,010 | 7,5 | 92,5 | 80 % Schweine-Gelatine 260 bloom | 30 % Pfefferminzaroma | 3380 | 25 sec |
| | | | | | | 10 % Glycerin | 70 % Pflanzenöl | | |
| | | | | | | 10 % Wasser* | | | |
| 6x | 5 | 70 | 0,014 | 7,5 | 92,5 | 70% Schweinegelatine 260 bloom | 15 % Pfefferminzaroma | 2160 | 45 sec |
| | | | | | | 20 % Glycerin | 85 % Pflanzenöl | | |
| | | | | | | 10 % Wasser* | | | |
| 7x | 5 | 55 | 0,011 | 7,5 | 92,5 | 70% Schweinegelatine 260 bloom | 15 % Pfefferminzaroma | 1820 | 15 sec |
| | | | | | | 20 % Glycerin | 85 % Pflanzenöl | | |
| | | | | | | 10 % Wasser* | | | |
| 8x | 5 | 64 | 0,013 | 9 | 91 | 70 % Schweine-Gelatine 260 bloom | 40% Pfefferminzaroma | 1880 | 40 sec |
| | | | | | | 20 % Glycerin | 60% Pflanzenöl | | |
| | | | | | | 10 % Wasser* | | | |
| | | | | | | 0,5 % Aspartam | | | |
| | | | | | | 0,5 % Acesulfam K | | | |
| | | | | | | 0,1 % Brilliantblau | | | |
| 9x | 5 | 73 | 0,015 | 12 | 88 | 70 % Schweine-Gelatine 260 bloom | 65% Mintaroma | 2670 | 40 sec |
| | | | | | | 20 % Glycerin | 35% Pflanzenöl | | |
| | | | | | | 10 % Wasser* | | | |
| | | | | | | 2,0 % Sucralose | | | |
| | | | | | | 0,1 % Brilliantblau | | | |
| 10x | 6 | 77 | 0,013 | 7,5 | 92,5 | 70% Schweinegelatine 260 bloom | 10 % Pfefferminzaroma | 1970 | 35 sec |
| | | | | | | 20 % Glycerin | 90 % Pflanzenöl | | |
| | | | | | | 10 % Wasser* | | | |
| | | | | | | 0,05 % Aspartam | | | |
| | | | | | | 0,05 % Acesulfam K | | | |
| 11x | 4 | 50 | 0,013 | 9 | 91 | 70% Schweinegelatine 260 bloom | 30 % Pfefferminzaroma | 1460 | 10 sec |
| | | | | | | 20 % Glycerin | 70 % Pflanzenöl | | |
| | | | | | | 10 % Wasser* | | | |
| | | | | | | 0,4 % Acesulfam K | | | |
| | | | | | | 0,1 % Brilliantblau | | | |
| 12x | 5 | 122 | 0,024 | 15 | 85 | 70% Schweinegelatine 260 bloom | 15 % Pfefferminzaroma | >6000 | 80 sec |
| | | | | | | 20 % Glycerin | 85 % Pflanzenöl | | |
| | | | | | | 10 % Wasser* | | | |
| 13x | 6 | 125 | 0,021 | 15 | 85 | 70% Schweinegelatine 260 bloom | 10 % Pfefferminzaroma | >6000 | 60 sec |
| | | | | | | 20 % Glycerin | 90 % Pflanzenöl | | |
| | | | | | | 10 % Wasser* | | | |
| 14x | 7 | 150 | 0,021 | 15 | 85 | 70% Schweinegelatine 260 bloom | 10 % Pfefferminzaroma | >6000 | 60 sec |
| | | | | | | 20 % Glycerin | 90 % Pflanzenöl | | |
| | | | | | | 10 % Wasser* | | | |
| 15x | 8 | 150 | 0,019 | 10 | 90 | 70% Schweinegelatine 260 bloom | 10 % Pfefferminzaroma | >6000 | 60 sec |
| | | | | | | 20 % Glycerin | 90 % Pflanzenöl | | |
| | | | | | | 10 % Wasser* | | | |
| 16x | 5 | 64 | 0,013 | 8 | 92 | 70% Schweinegelatine 260 bloom | 15 % Pfefferminzaroma | 2180 | 30 sec |
| | | | | | | 20 % Glycerin | 85 % Pflanzenöl | | |
| | | | | | | 10 % Wasser* | | | |
| | | | | | | 0,05 % Aspartam | | | |
| | | | | | | 0,05 % Acesulfam K | | | |
| 17x | 5 | 65 | 0,013 | 7,5 | 92,5 | 70% Schweinegelatine 260 bloom | 15 % Pfefferminzaroma | 1550 | 30 sec |
| | | | | | | 20 % Propylenglycol | 85 % Pflanzenöl | | |
| | | | | | | 10% Wasser* | | | |
| 18x | 5 | 55 | 0,01 | 7,5 | 92,5 | 60% Schweinegelatine 260 bloom | 15 % Pfefferminzaroma | 900 | 25 sec |
| | | | | | | 30 % Propylenglycol | 85 % Pflanzenöl | | |
| | | | | | | 10 % Wasser* | | | |
| 19x | 5 | 72 | 0,014 | 9 | 91 | 80% Schweinegelatine 260 bloom | 15 % Pfeffermlnzaroma | 4000 | 40 sec |
| | | | | | | 10 % Sorbitol | 85 % Pflanzenöl | | |
| | | | | | | 10 % Wasser* | | | |

**Effekt von Hydrokolloiden**

| Nr. | Kapsel durchmesser | Hülldicke | Verhältnis Hülldicke/ Kapseldurchmesser | Anteil Hülle | Anteil Kern | Zusammensetzung Hülle | Zusammensetzung Kern | Härte | Auflösen im Mund |
|---|---|---|---|---|---|---|---|---|---|
| | [mm] | [µm] | | [Gew. %] | [Gew.%] | | | [g] | [sec] |
| 20x | 5 | 68 | 0,014 | 9 | 91 | 69% Schweinegelatine 260 bloom | 50 % Pfefferminzaroma | nicht | 60 sec |
| | | | | | | 20 % Propylenglykol | 50 % Pflanzenöl | bestimmt | |
| | | | | | | 10 % Wasser* | | | |
| | | | | | | 1 % Gellan Gum KELCOGEL F | | | |
| 21x | 5 | 75 | 0,015 | 12 | 88 | 69% Schweinegelatine 260 bloom | 50 % Pfefferminzaroma | nicht | 75 sec |
| | | | | | | 20 % Glycerin | 50 % Pflanzenöl | bestimmt | |
| | | | | | | 10 % Wasser* | | | |
| | | | | | | 0,8 % Gellan Gum KELCOGEL F | | | |
| 22x | 5 | 70 | 0,014 | 9 | 91 | 67% Schweinegelatine 260 bloom | 50 % Pfefferminzaroma | nicht | 50 sec |
| | | | | | | 10 % Glycerin | 50 % Pflanzenöl | bestimmt | |
| | | | | | | 10 % Wasser* | | | |
| | | | | | | 2 % Gellan Gum KELCOGEL F | | | |

Gellan Gum kann von Kelco, einer Division von Merck&Co. unter dem Handlesnamen KELCOGEL F bezogen werden

Bei Formulierungen mit Gellan Gum soll die Hüllmischung auf 80°C erhitzt werden, bis eine klare Lösung entstanden ist. Eine Auskühlung der Flüssigkeitsoberfläche soll vermieden werden.

**Gelatinearten**

| Nr. | Kapsel durch-messer | Hüll-dicke | Verhältnis Hülldicke/ Kapsel-durch-messer | Anteil Hülle | Anteil Kern | Zusammensetzung Hülle | Zusammensetzung Kern | Härte | Auflösen im Mund |
|---|---|---|---|---|---|---|---|---|---|
| | [mm] | [µm] | | [Gew. %] | [Gew.%] | | | [g] | [sec] |
| 23x | 5 | 73 | 0,015 | 11 | 89 | 70 % Schweine-Gelatine 260 bloom | 65% Mintaroma | 2670 | 40 sec |
| | | | | | | 20 % Glycerin | 35% Pflanzenöl | | |
| | | | | | | 10% Wasser* | | | |
| | | | | | | 2,0 % Sucralose | | | |
| | | | | | | 0,1 % Brilliantblau | | | |
| 24x | 5 | 60 | 0,012 | 7,5 | 92,5 | 70 % Rinder-Gelatine 240 bloom | 30 % Zimtaroma | 2150 | 45 sec |
| | | | | | | 20 % Glycerin | 70 % Pflanzenöl | | |
| | | | | | | 10 % Wasser* | | | |
| | | | | | | 0,2 % Allurarot | | | |
| 25x | 5 | 66 | 0,013 | 11 | 89 | 70 % Hühner-Gelatine 250 bloom | 65% Mintaroma | 3800 | 35 sec |
| | | | | | | 20 % Glycerin | 35% Pflanzenöl | | |
| | | | | | | 10 % Wasser* | | | |
| | | | | | | 2,0 % Sucralose | | | |
| | | | | | | 0,1 % Brilliantblau | | | |
| 26 | 5 | 94 | 0,019 | 11 | 89 | 55 % Schweine-Gelatine 260 bloom | 65% Mintaroma | 3240 | 25 sec |
| | | | | | | 17 % Fisch Gelatine 0 bloom | 35% Pflanzenöl | | |
| | | | | | | 18 % Glycerin | | | |
| | | | | | | 10 % Wasser* | | | |
| 27 | 5 | 80 | 0,016 | 11 | 89 | 55 % Hühner-Gelatine 260 bloom | 65% Mintaroma | 3520 | 30 sec |
| | | | | | | 17 % Fisch Gelatine 0 bloom | 35% Pflanzenöl | | |
| | | | | | | 18 % Glycerin | | | |
| | | | | | | 10 % Wasser* | | | |
| 28 | 5 | 75 | 0,015 | 11 | 89 | 35 % Hühner-Gelatine 250 bloom | 65% Mintaroma | 3000 | 25 sec |
| | | | | | | 35 % Fisch Gelatine 110 bloom | 35% Pflanzenöl | | |
| | | | | | | 20 % Glycerin | | | |
| | | | | | | 10 % Wasser* | | | |
| 29x | 5 | 72 | 0,014 | 11 | 89 | 70 % Fisch-Gelatine 165 bloom | 65% Mintaroma | 2500 | 30 sec |
| | | | | | | 20 % Glycerin | 35% Pflanzenöl | | |
| | | | | | | 10% Wasser* | | | |
| | | | | | | 2 % Gellan Gum KELCOGEL F | | | |
| | | | | | | 2,0 % Sucralose | | | |
| | | | | | | 0,1 % Brilliantblau | | | |

| | | | | | | | | | |
|---|---|---|---|---|---|---|---|---|---|
| *Bemerkung: Anhaftendes Restwasser hängt von Umgebungsfeuchte ab und kann daher schwanken x : Vergleichsbeispiele | | | | | | | | | |

**Tabelle: Gelierpunkte**

| | Zusammensetzung der wäßrigen Hüllösung | Gelier punkt | Viskosität bei 80°C | Bemerkung | Flexibilität * |
|---|---|---|---|---|---|
| Ax | 75 % Wasser | 29°C | 54 mPas | | gering |
| | 20 % Schweinegelatine 260 bloom | | | | |
| | 5 % Glycerin | | | | |
| Bx | 75 % Wasser | 34°C | 61 mPas | | gering |
| | 20 % Rindergelatine 240 bloom | | | | |
| | 5 % Glycerin | | | | |
| Cx | 75 % Wasser | 35°C | 71 mPas | | gering |
| | 20 % Hühnergelatine 250 bloom | | | | |
| | 5 % Glycerin | | | | |
| D | 75 % Wasser | 30°C | 130 mPas | | mittel |
| | 10 % Schweinegelatine 260 bloom | | | | |
| | 10 % Fischgelatine 110 bloom | | | | |
| | 5 % Glycerin | | | | |
| E | 75 % Wasser | 29°C | 90 mPas | | hoch |
| | 15 % Schweinegelatine 260 bloom | | | | |
| | 5 % Fischgelatine 0 bloom | | | | |
| | 5 % Glycerin | | | | |
| F | 75 % Wasser | 32°C | 85 mPas | | hoch |
| | 15 % Hühnergelatine 250 bloom | | | | |
| | 5 % Fischgelatine 0 bloom | | | | |
| | 5 % Glycerin | | | | |
| Gx | 75 % Wasser | 50°C | 300 mPas | | gering |
| | 19,6 % Fischgelatine 165 bloom | | | | |
| | 0,4 % Gellan Gum Kelcogel F | | | | |
| | 5 % Glycerin | | | | |
| Hx | 75 % Wasser | 20°C | 42 mPas | feuchte Kapselhülle zu instabil für weitere Prozeßschritte | mittel |
| | 20 % Fischgelatine 110 bloom | | | | |
| | 5 % Glycerin | | | | |
| | | | | | |
| | | | | | |
| Ix | 75 % Wasser | 24°C | 72 mPas | feuchte Kapselhülle zu instabil für weitere Prozeßschritte | mittel |
| | 20 % Fischgelatine 165 bloom | | | | |
| | 5 % Glycerin | | | | |
| | | | | | |
| | | | | | |
| Kx | 75 % Wasser | 33°C | 90 mPas | | gering |
| | 20 % Schweinegelatine 80 bloom | | | | |
| | 5 % Glycerin | | | | |

| | | | | | |
|---|---|---|---|---|---|
| * Flexibilität: Beurteilung beim Umknicken eines 200 µm dicken Films, der aus der Hülllösung durch Ausgießen und Trocknen an der Luft (20°C, 40 % relative Luftfeuchte, mindestens 20 h) hergestellt wurde x : Vergleichsbeispiele | | | | | |

## Patentansprüche

1. Sphärische Kapsel mit einem flüssigen Kern und einer diesen Kern umgebenden nahtlosen, festen Hülle, wobei
- der Durchmesser der Kapsel im Bereich von 4 - 8 mm liegt,
- die Dicke der Hülle im Bereich von 20 - 200 µm liegt,
- das Verhältnis von Hülldicke zu Kapseldurchmesser im Bereich von 0,004 - 0,04 liegt,
- die Hülle 70 - 90 Gew.-% Gelatine und 10 - 30 Gew.-% Weichmacher enthält, bezogen auf die Trockenmasse der Hülle, und
- der Kern einen Aromaanteil im Bereich von 1 - 100 Gew.-% enthält, bezogen auf die Gesamtmasse des Kerns, wobei zur Herstellung der Hülle (a) eine Gelatine mit einem Bloomwert von mindestens 200 und zusätzlich (b) eine Gelatine mit Bloomwert 0 und/oder Fischgelatine mit einem Bloomwert < 200 eingesetzt wird.

2. Kapsel nach Anspruch 1, **dadurch gekennzeichnet, dass**
- der Durchmesser der Kapsel im Bereich von 4,5 - 6,5 mm, bevorzugt 4,5 - 5,5 mm,
- die Dicke der Hülle im Bereich von 50 - 150 µm, bevorzugt 50 - 90 µm,
- das Verhältnis von Hülldicke zu Kapseldurchmesser im Bereich von 0,01 - 0,03, bevorzugt 0,01 - 0,02;
liegt.

3. Kapsel nach einem der vorangehenden Ansprüche, **dadurch gekennzeichnet, dass** die Hülle aus einer Gelatine und Weichmacher umfassenden Mischung hergestellt ist, die einen Gelierpunkt im Bereich zwischen 15°C und 60°C, vorzugsweise zwischen 20°C und 40°C, besonders bevorzugt zwischen 25°C und 35°C besitzt.

4. Kapsel nach einem der vorangehenden Ansprüche, wobei die Fischgelatine eine Kaltwasser-Fischgelatine ist und/oder einen Gelierpunkt < 20°C besitzt.

5. Kapsel nach einem der vorangehenden Ansprüche, **dadurch gekennzeichnet, daß** der flüssige Kern einen Süßstoff enthält, der aus der Gruppe ausgewählt ist, die aus Thaumatin, Neohesperidin, Miraculin und deren Mischungen besteht.

6. Kapsel nach einem der vorangehenden Ansprüche, **dadurch gekennzeichnet, daß** die Konzentration des Weichmachers in der Hülle, 10 - 30 Gew.-%, bevorzugt 15 - 20 Gew.-% beträgt, bezogen auf die Gesamttrockenmasse der Hülle.

7. Kapsel nach einem der vorangehenden Ansprüche, **dadurch gekennzeichnet, daß** der Weichmacher ein oder mehrere Polyole umfasst, vorzugsweise ausgewählt aus der Gruppe, die aus Glycerin, Propylenglykol, Sorbitol und Maltitol besteht.

8. Kapsel nach einem der vorangehenden Ansprüche, **dadurch gekennzeichnet, daß** die Gelatine ausgewählt ist aus der Gruppe, die aus Schweinegelatine, Rindergelatine, Hühnergelatine, Fischgelatine und deren Mischungen besteht.

9. Kapsel nach einem der vorangehenden Ansprüche, **dadurch gekennzeichnet, daß** die Hülle einen Süßstoff enthält, der vorzugsweise aus der Gruppe ausgewählt ist, die aus Sucralose, Aspartam, Acesulfam K, Thaumatin, Na-Saccharin , Neohesperidin und deren Mischungen besteht.

10. Kapsel nach einem der vorangehenden Ansprüche, **dadurch gekennzeichnet, daß** die Hülle Gellan Gum enthält.

11. Kapsel nach einem der vorangehenden Ansprüche, **dadurch gekennzeichnet, daß** die Hülle 0,4 - 3 Gew.-% Gellan Gum enthält, bezogen auf die Trockenmasse der Hülle.

12. Verfahren zur Herstellung einer Kapsel nach einem der vorangehenden Ansprüche, **dadurch gekennzeichnet, dass** ein flüssiges Kernmaterial und eine gelatinehaltige aushärtbare Hüllmischung gleichzeitig durch eine konzentrische Mehrstoffdüse gepumpt werden, so dass sie unter Kapselbildung in eine Kühlflüssigkeit eintropfen.

13. Aushärtbare Hüllmischung zur Verwendung bei der Herstellung einer Kapsel nach einem der Ansprüche 1-11, **dadurch gekennzeichnet, dass** die Hüllmischung Gelatine und Weichmacher umfasst.

14. Verwendung einer Mischung aus (a) einer Gelatine mit einem Bloomwert von mindestens 200 und (b) einer Gelatine mit Bloomwert 0 und/oder einer Fischgelatine, zur Einstellung von Härte und Auflösevermögen der Hülle einer sphärischen Kapsel mit einem flüssigen Kern und einer diesen Kern umgebenden nahtlosen, festen Hülle.

## Claims

1. A spherical capsule with a liquid core and a seamless, solid shell surrounding this core, wherein
- the diameter of the capsule is in the range of 4 - 8 mm,
- the thickness of the shell is in the range of 20 - 200 µm,
- the ratio of shell thickness to capsule diameter is in the range of 0.004 - 0.04,
- the shell contains 70 - 90 wt.% gelatin and 10 - 30 wt.% plasticiser, based on the dry mass of the shell, and
- the core contains a proportion of flavouring in the range of 1 - 100 wt.%, based on the total mass of the core, wherein, to produce the shell, (a) a gelatin with a bloom value of at least 200 and additionally (b) a gelatin with a bloom value of 0 and/or fish gelatin with a bloom value of < 200 is used.

2. The capsule according to claim 1, **characterised in that**
- the diameter of the capsule is in the range of 4.5 - 6.5 mm, preferably 4.5 - 5.5 mm,
- the thickness of the shell is in the range of 50 - 150 µm, preferably 50 - 90 µm,
- the ratio of shell thickness to capsule diameter is in the range of 0.01 - 0.03, preferably 0.01 - 0.02.

3. The capsule according to one of the preceding claims, **characterised in that** the shell is produced from a mixture comprising gelatin and plasticiser having a gel point in the range of between 15°C and 60°C, preferably between 20°C and 40°C, particularly preferably between 25°C and 35°C.

4. The capsule according to one of the preceding claims, wherein the fish gelatin is a cold-water fish gelatin and/or has a gel point of < 20°C.

5. The capsule according to one of the preceding claims, **characterised in that** the liquid core contains a sweetener selected from the group consisting of thaumatin, neohesperidin, miraculin and mixtures thereof.

6. The capsule according to one of the preceding claims, **characterised in that** the concentration of the plasticiser in the shell is 10 - 30 wt.%, preferably 15 - 20 wt.%, based on the total dry mass of the shell.

7. The capsule according to one of the preceding claims, **characterised in that** the plasticiser comprises one or more polyols, preferably selected from the group consisting of glycerol, propylene glycol, sorbitol and maltitol.

8. The capsule according to one of the preceding claims, **characterised in that** the gelatin is selected from the group consisting of pork gelatin, beef gelatin, chicken gelatin, fish gelatin and mixtures thereof.

9. The capsule according to one of the preceding claims, **characterised in that** the shell contains a sweetener, which is preferably selected from the group consisting of sucralose, aspartame, acesulfame K, thaumatin, Na-saccharin, neohesperidin and mixtures thereof.

10. The capsule according to one of the preceding claims, **characterised in that** the shell contains gellan gum.

11. The capsule according to one of the preceding claims, **characterised in that** the shell contains 0.4 - 3 wt.% gellan gum, based on the dry mass of the shell.

12. A process for the production of a capsule according to one of the preceding claims, **characterised in that** a liquid core material and a gelatin-containing, hardenable shell mixture are simultaneously pumped through a concentric multiple nozzle so that they drop into a cooling liquid, forming a capsule.

13. A hardenable shell mixture for use in the production of a capsule according to one of claims 1-11, **characterised in that** the shell mixture comprises gelatin and plasticiser.

14. The use of a mixture of (a) a gelatin with a bloom value of at least 200 and (b) a gelatin with a bloom value of 0 and/or a fish gelatin to adjust the hardness and dissolving properties of the shell of a spherical capsule with a liquid core and a seamless, solid shell surrounding this core.

## Revendications

1. Capsule sphérique ayant un noyau liquide et une enveloppe solide sans soudure entourant ce noyau,
- le diamètre de la capsule se situant dans le domaine de 4-8 mm,
- l'épaisseur de l'enveloppe se situant dans le domaine de 20-200 µm,
- le rapport de l'épaisseur de l'enveloppe au diamètre de la capsule se situant dans le domaine de 0,004 - 0,04,
- l'enveloppe contenant 70-90 % en masse de gélatine et 10-30 % en masse de plastifiant, par rapport à la masse sèche de l'enveloppe, et
- le noyau ayant une teneur en arômes se situant dans le domaine de 1-100 % en masse par rapport à la masse totale du noyau, et on utilise pour la préparation de l'enveloppe (a) une gélatine ayant un degré Bloom d'au moins 200 et, en plus, (b) une gélatine ayant un degré Bloom de 0 et/ou une gélatine de poisson ayant un degré Bloom < 200.

2. Capsule selon la revendication 1, **caractérisée en ce que**
- le diamètre de la capsule se situe dans le domaine de 4,5-6,5 mm, de préférence de 4,5-5,5 mm,
- l'épaisseur de l'enveloppe se situe dans le domaine de 50-150 µm, de préférence de 50-90 µm,
- le rapport de l'épaisseur de l'enveloppe au diamètre de la capsule se situe dans le domaine de 0,01-0,03, de préférence de 0,01-0,02.

3. Capsule selon l'une des revendications précédentes, **caractérisée en ce que** l'enveloppe est préparée à partir d'un mélange comprenant de la gélatine et un plastifiant qui possède un point de gélification se situant dans le domaine entre 15°C et 60°C, de préférence entre 20°C et 40°C, de façon particulièrement préférée entre 25°C et 35°C.

4. Capsule selon l'une des revendications précédentes, dans laquelle la gélatine de poisson est une gélatine de poisson des eaux froides et/ou possède un point de gélification < 20°C.

5. Capsule selon l'une des revendications précédentes, **caractérisée en ce que** le noyau liquide contient un édulcorant qui est choisi dans le groupe constitué par la thaumatine, la néohespéridine, la miraculine et leurs mélanges.

6. Capsule selon l'une des revendications précédentes, **caractérisée en ce que** la concentration du plastifiant dans l'enveloppe est de 10-30 % en masse, de préférence de 15-20 % en masse, par rapport à la masse sèche totale de l'enveloppe.

7. Capsule selon l'une des revendications précédentes, **caractérisée en ce que** le plastifiant comprend un ou plusieurs polyols, de préférence choisis dans le groupe constitué par le glycérol, le propylèneglycol, le sorbitol et le maltitol.

8. Capsule selon l'une des revendications précédentes, **caractérisée en ce que** la gélatine est choisie dans le groupe constitué par la gélatine de porc, la gélatine de boeuf, la gélatine de poulet, la gélatine de poisson et leurs mélanges.

9. Capsule selon l'une des revendications précédentes, **caractérisée en ce que** l'enveloppe contient un édulcorant qui est de préférence choisi dans le groupe constitué par le sucralose, l'aspartame, l'acésulfame K, la thaumatine, la saccharine sodique, la néohespéridine et leurs mélanges.

10. Capsule selon l'une des revendications précédentes" **caractérisée en ce que** l'enveloppe contient de la gomme gellane.

11. Capsule selon l'une des revendications précédentes, **caractérisée en ce que** l'enveloppe contient 0,4-3 % en masse de gomme gellane par rapport à la masse sèche de l'enveloppe.

12. Procédé de préparation d'une capsule selon l'une des revendications précédentes, **caractérisé en ce que** l'on pompe en même temps un matériau de noyau et un mélange d'enveloppe durcissable contenant de la gélatine à travers un injecteur à plusieurs composants concentrique, de façon qu'ils pénètrent goutte à goutte dans un liquide de refroidissement en formant des capsules.

13. Mélange d'enveloppe durcissable à utiliser dans la préparation d'une capsule selon l'une des revendications 1-11, **caractérisé en ce que** le mélange d'enveloppe comprend de la gélatine et un plastifiant.

14. Utilisation d'un mélange de (a) une gélatine ayant un degré Bloom d'au moins 200 et de (b) une gélatine ayant un degré Bloom de 0 et/ou une gélatine de poisson, pour le réglage de la dureté et de la capacité de dissolution de l'enveloppe d'une capsule sphérique ayant un noyau liquide et une enveloppe solide sans soudure entourant ce noyau.
